(19) Europäisches Patentamt
European Patent Office
Office européen des brevets

(11) Veröffentlichungsnummer: **0 011 845**
**A2**

(12) **EUROPÄISCHE PATENTANMELDUNG**

(21) Anmeldenummer: **79104695.6**

(22) Anmeldetag: **26.11.79**

(51) Int. Cl.$^3$: **A 61 K 7/06**

(30) Priorität: **30.11.78 DE 2851831**

(43) Veröffentlichungstag der Anmeldung: **11.06.80 Patentblatt 80/12**

(84) Benannte Vertragsstaaten: **BE CH DE FR GB IT NL SE**

(71) Anmelder: **Henkel Kommanditgesellschaft auf Aktien
Postfach 1100 Henkelstrasse 67
D-4000 Düsseldorf 1(DE)**

(72) Erfinder: **Möller, Hinrich Dr.
Erlanger Strasse 45
D-4000 Düsseldorf 13(DE)**

(72) Erfinder: **Thimm, Hans-Joachim Dr.
Kantstrasse 4 - 6
D-4010 Hilden(DE)**

(54) **Haarbehandlungsmittel mit Antischuppenwirkung.**

(57) Haarbehandlungsmittel mit Antischuppenwirkung mit einem Gehalt an Alkoxy-benzoyl-aminoalkansäuren der Formel

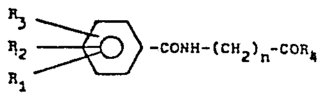

$R_1$ = Alkoxygruppe mit 1 - 4 C-Atomen in 3-oder 4-Stellung.
$R_2, R_3$ = H, OH, Alkyl- oder Alkoxygruppe mit 1 - 4 C-Atomen.
$R_4$ = OH, Alkoxy- oder gegebenenfalls substituierte Aminogruppe.
n = 1-6.

EP 0 011 845 A2

Croydon Printing Company Ltd.

4000 Düsseldorf, den 29.11.1978
Henkelstraße 67

0011845
HENKEL KGaA
ZR-FE/Patente
Z/Br

# P a t e n t a n m e l d u n g

D 5881

"Haarbehandlungsmittel mit Antischuppenwirkung"

Gegenstand der Erfindung sind Haarbehandlungsmittel mit Antischuppenwirkung, die als Wirkstoffe Alkoxy-benzoyl-aminoalkansäuren bzw. deren Derivate enthalten.

Haarbehandlungsmittel zur Beseitigung unerwünschter Schuppenbildung auf der Kopfhaut sind bereits in größerer Anzahl auf Basis der unterschiedlichsten Wirkstoffe und Zubereitungen bekannt. Einen bevorzugten Platz nehmen dabei die Haarbehandlungsmittel auf der Grundlage wäßriger Tensidlösungen wie Shampoos und Haarkurspülungen ein. Um als brauchbare Antischuppenmittel eingesetzt werden zu können, müssen derartige Produkte eine Reihe von Voraussetzungen erfüllen, die nachfolgend kurz aufgeführt werden.

Die Wirkstoffe müssen toxikologisch und dermatologisch unbedenklich sein und auch die damit hergestellten Haarbehandlungsmittel müssen sich durch eine gute Hautverträglichkeit auszeichnen. Die Antischuppenwirksamkeit der Kompositionen darf auch durch eine lange Lagerung, z.B. durch Reaktion mit anderen Bestandteilen des

/2

Mittels, nicht ungünstig beeinflußt werden. Die Wirkung des Mittels darf nicht zu schnell abklingen, sondern sie muß zumindest die Zeit zwischen zwei Behandlungen, seien es nun Haarwäschen oder andere Haarbehandlungen mit dem Antischuppenwirkstoff, überbrücken. Ferner soll der Antischuppenwirkstoff eine gute Verträglichkeit mit den üblicherweise verwendeten anderen Bestandteilen der Haarbehandlungsmittel aufweisen und im Hinblick auf Aussehen und Geruch ansprechend wirken.

Es wurde nun gefunden, daß diese Forderungen weitgehend erfüllt werden, wenn man Haarbehandlungsmittel mit Antischuppenwirkung verwendet, die Alkoxy-benzoylaminoalkansäuren bzw. deren Derivate der allgemeinen Formel

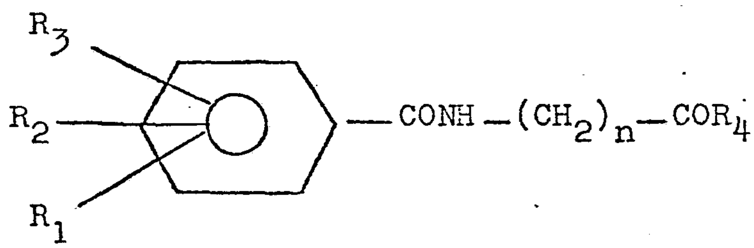

in der $R_1$ für eine Alkoxygruppe mit 1 - 4 C-Atomen, vorzugsweise eine Methoxygruppe in der 3- oder 4- Position, $R_2$ und $R_3$ unabhängig voneinander für ein Wasserstoffatom, einen Alkylrest oder eine Alkoxygruppe mit 1 - 4 C-Atomen oder eine Hydroxygruppe, $R_4$ für eine Hydroxygruppe, eine Alkoxygruppe oder eine gegebenenfalls substituierte Aminogruppe und n für die Zahlen 1 - 6 stehen, in einer Menge von 0,01 bis 5 Gewichtsprozent, vorzugsweise 0,5 bis 3 Gewichtsprozent, bezogen auf das gesamte Mittel, enthalten.

Besonders günstige schuppenhemmende Effekte weisen Haarbehandlungsmittel auf, die neben den erfindungsgemäß einzusetzenden Alkoxy-benzoylaminoalkansäuren bzw. deren Derivaten Fettalkohol-polyalkylenoxid-Addukte, vorzugsweise solche von Fettalkoholen der Kettenlängen mit

12 - 18 Kohlenstoffatomen mit 5 - 40 Mol Ethylenoxid-Einheiten, enthalten.

Die in den erfindungsgemäßen Haarbehandlungsmitteln mit Antischuppenwirkung einzusetzenden Alkoxy-benzoylaminoalkansäuren bzw. deren Salze sind bekannte Verbindungen oder nach üblichen Verfahren der organischen Synthese herstellbar. So können im allgemeinen die Ester der Aminoalkansäuren leicht mit den entsprechenden Benzoesäurechloriden acyliert werden. Die Ester können dann nach allgemein üblichen Verfahren in die freien Säuren oder deren Salze bzw. in die Amide überführt werden.

Als in den erfindungsgemäßen Haarbehandlungsmitteln mit Antischuppenwirkung einzusetzende Alkoxy-benzoyl-aminoalkansäuren bzw. deren Derivate sind zum Beispiel N-(3-Methoxybenzoyl)-, N-(3-Methoxy,4-methyl-benzoyl)-, N-(5-Methoxy,2-methyl-benzoyl)-, N-(3-Methoxy, 4-hydroxy-benzoyl)-, N-(3,4-Dimethoxy-benzoyl)-, N-(3,5-Dimethoxy-benzoyl)-, N-(2,5-Diethoxy-benzoyl)-, N-(3,4,5-Trimethoxy-benzoyl)-glycin,-glycinethylester, -glycinamid, -β-alanin, -4-aminobuttersäurepropylester, -5-aminovaleriansäure, -5-aminovaleriansäureethylester, -5-aminovaleriansäure-diethylamid, -6-amino-capronsäure-ethylester, -6-amino-capronsäure-butylamid, -6-amino-capronsäure-diethylamid zu nennen.

Für den Fall des Einsatzes der Alkoxy-benzoylaminoalkansäuren werden diese zuvor mit toxikologisch unbedenklichen Basen auf einen pH-Wert zwischen 5 und 7,5 eingestellt. Bevorzugte Basen werden dabei Natronlauge, Ammoniak und Triethanolamin sein.

Als erfindungsgemäße Haarbehandlungsmittel mit Antischuppenwirkung sind alle Arten der bisher für solche Zwecke verwendeten Mittel geeignet, wie z.B. Haarwasser, Frisiercremes, Haarwaschmittel, Haarfestiger,

Haarkuren, Haarspülungen, Haarlotionen auf Basis von Wasser-in-Öl und Öl-in-Wasser-Emulsionen. Besondere Bedeutung kommt dabei den Haarwaschmitteln, Haarkuren und Haarspülungen zu. Derartige Produkte enthalten neben den 0,01 - 5 Gew.-%, vorzugsweise 0,5 - 3 Gew.-%, an Alkoxy-benzoylaminoalkansäuren bzw. deren Derivaten die darin üblichen Bestandteile in den gebräuchlichen Mengen.

Die erfindungsgemäßen Haarbehandlungsmittel können im einfachsten Fall aus einer wäßrigen oder wäßrig-alkoholischen Lösung beziehungsweise Dispersion des Wirkstoffes nebst Parfüm und Farbstoff bestehen und in dieser Form als Haarwasser oder Nachspülmittel eingesetzt werden. Die Mittel können in Anpassung an andere Verwendungszwecke, wie z.B. Haarshampoos, Haarkuren usw., weitere in derartigen Mitteln gebräuchliche Komponenten in üblichen Mengenverhältnissen enthalten.

Demgemäß können als tensidische Bestandteile Sulfattenside wie Fettalkoholsulfate, Fettalkoholethersulfate mit 3 bis 4 Ethylenoxideinheiten im Molekül, Alkylphenolethersulfate, Monoglyceridsulfate, ferner Fettsäure-Eiweißkondensationsprodukte, Fettsäuresarcoside und Fettsäuremethyltauride eingesetzt werden. Ferner kommen auch amphotere Tenside, wie z.B. die unter der Bezeichnung Miranole bekannten Imidazol-Abkömmlinge in Betracht. Die vorgenannten anionischen Tenside liegen insbesondere in Form ihrer Natrium- und Triethanolaminsalze, in Einzelfällen, wie z.B. Myristylalkoholethersulfat, auch in Form der Magnesiumsalze vor. Besonders günstige schuppenhemmende Effekte werden erzielt, wenn als tensidische Komponente Fettalkoholpolyalkylenoxid-Addukte, vorzugsweise solche aus Fettalkoholen mit 12 - 18 Kohlenstoffatomen mit 5 - 40 Ethylenoxid-Einheiten, in Kombination mit den Alkoxy-benzoylaminoalkansäuren

bzw. deren Derivaten und gegebenenfalls anderen oben genannten Tensiden eingesetzt werden.

Als emulgierend wirkende Bestandteile können in den erfindungsgemäßen Mitteln insbesondere Seifen der Stearin-, Laurin- und Ölsäure in Form ihrer Natrium-, Kalium- oder Alkanolaminsalze vorhanden sein, ferner die oben bereits genannten Sulfattenside, Polyol-Fettsäureester, z.B. Glycerinmonostearat, Propylenglykolmonostearat, Diethylenglykolmonostearat, zum Teil im Gemisch mit anionaktiven Emulgatoren, Fettalkoholgemische in Kombination mit anionaktiven Emulgatoren; nichtionische Emulgatoren wie Polyethylenoxidester, z.B. Polyoxyethylenstearat, -oleat usw., Polyethylenoxidsorbitanester, einfache Sorbitanester wie Sorbitanmonolaurat, -oleat, -sesquioleat, Sterine, Polyethylenglykolester, wie z.B. die Mono- und Dilaurate, -oleate oder -stearate von Polyethylenglykol mit Molgewichten von 200 bis 600; kationaktive Emulgatoren wie Laurylammoniumchlorid, Cetylpyridiniumchlorid, Cetyltrimethylammoniumbromid, Diisobutylphenoxyethoxyethyldimethylbenzylammoniumchlorid, Alkyldimethylbenzylammoniumchlorid mit 10 - 14 C-Atomen im Alkylrest, N-(Stearylcolaminoformylmethyl)-Pyridiniumchlorid o.ä.; Beispiele für ampholytische Emulgatoren sind Ethylencycloimido-1-lauryl-2-hydroxyethylennatriumalkoholat, Triethanolamin-β-alanin, N-Lauryl-aminopropionat, N-Lauryl-iminodipropionat, N-Lauryl-Diethyltriaminoessigsäure.

Als gegebenenfalls zuzusetzende Verdickungsmittel können ebenfalls übliche Substanzen genommen werden, wie z.B. insbesondere Natriumalginatschleime, Fettsäurealkylolamide und zum Teil Tyloseschleime, ferner höhermolekulare Polyethylenglykolmono- oder diester von Fettsäuren, insbesondere der Stearin- und Laurinsäure. Als Verdickungsmittel kommen in einzelnen Fällen auch Elektrolyte wie Kochsalz oder Ammoniumchlorid in Kombination mit Alkylethersulfaten in Betracht.

Den erfindungsgemäßen Mitteln können in bestimmten Fällen ferner Überfettungsmittel zugesetzt werden, beispielsweise polyoxyethylierte Lanolinderivate, Lecithinderivate oder die bereits oben erwähnten Alkylolamide, denen eine gewisse auffettende Wirkung zukommt. Letztere können auch als Schaumstabilisatoren in Shampoos dienen.

Die erfindungsgemäßen Mittel können ferner sogenannte Gerüstbestandteile wie Paraffin, Fettstoffe, Lanolin und Wollfettalkohole, biogene Wirkstoffe wie Pflanzenextrakte, Eiweißabbauprodukte und Vitaminkomplexe, Lösungsvermittler wie niedere Glykole, z.B. 1,3-Propandiol, 1,3-Butylenglykol, Diethylenglykol, Filmbildner wie Polyvinylpyrrolidon, Vinylpyrrolidon-Vinylacetat-Copolymerisate, Polymere der Acrylsäurereihe, Dimethylhydantoin-Formaldehydharze, Polymere der 2-Alkyl-2-oxazolin-Reihe enthalten.

Die nachfolgenden Beispiele sollen den Gegenstand vorliegender Erfindung näher erläutern, ohne ihn jedoch hierauf zu beschränken.

## B e i s p i e l e

Nachstehend werden zunächst Herstellungsbeispiele für erfindungsgemäß einzusetzende Alkoxy-benzoylaminoalkansäuren bzw. deren Derivate aufgeführt.

A) N-(3,4,5-Trimethoxy-benzoyl)-6-amino-capronsäure-ethylester.

Zu einer Lösung von 30,0 g (0,189 Mol) 6-Aminocapronsäureethylester und 19,1 g (0,189 Mol) Triethylamin in 300 ml Toluol wurden unter Rühren und Kühlen portionsweise 43,5 g (0,189 Mol) 3,4,5-Trimethoxybenzoylchlorid gegeben. Nach einstündigem Rühren bei Raumtemperatur und Erwärmen auf 80° C wurde nach einer weiteren Stunde abgekühlt und der Niederschlag abfiltriert. Der Niederschlag wurde mit $CH_2Cl_2$ bei Raumtemperatur extrahiert, die Lösung mit der Toluolphase vereinigt und zusammen eingedampft. Der Rückstand wurde aus $CH_2Cl_2$/Petrolether umkristallisiert. Es wurden 37 g, das sind 56 % der Theorie, N-(3,4,5-Trimethoxy-benzoyl)-6-amino-capronsäure-ethylester vom Schmelzpunkt 82 - 84° C erhalten.

B) N-(3,4,5-Trimethoxy-benzoyl)-6-amino-capronsäure

Die Verbindung wurde durch Hydrolyse von N-(3,4,5-Trimethoxy-benzoyl)-6-amino-capronsäure-ethylester mit verdünnter wässrig-alkoholischer Natronlauge erhalten und besitzt einen Schmelzpunkt von 120 - 124° C.

Die Prüfung auf Antischuppenwirkung wurde in folgender Weise durchgeführt, wobei B) als mit Natronlauge auf einen pH-Wert von 6 neutralisiertes Produkt zum Einsatz gelangte:

Die Prüfsubstanzen wurden in 5 %iger Konzentration zusammen mit 5 % Ölsäure in 70 %igem Ethanol gelöst und in alternierender Reihenfolge und in rotierendem Schema auf den Rücken junger, geschorener weiblicher Meerschweinchen im Gewicht von 600 ± 50 g aufgetragen. Die Einwirkungszeit der Substanzen wurde dabei auf 60 Minuten begrenzt, da diese Zeit ausreichte, um eine Wirkung zu erzielen. Anschließend wurden die Tiere mit warmem Wasser 30 Sekunden lang abgeduscht. Die Reihenfolge der Tiere bei der Applikation wechselte dabei täglich, um Differenzen in der Einwirkungszeit zwischen dem ersten und letzten Tier der Gruppe auf ein Minimum zu begrenzen. Diese Behandlung geschah an vier aufeinanderfolgenden Tagen, darauf folgte eine Ruhezeit (Entwicklungszeit von drei Tagen). Am siebenten Tag wurde ausgewertet, und zwar a) kutimetrisch auf Hautfaltendicke instrumentell mit dem Cutimeter nach Götze und b) visuell auf Schuppenbildung bzw. Hemmung nach einer 4-Punkte-Skala folgender Definition:

0 = keine Schuppen
1 = leichte Schuppen
2 = kräftige Schuppen
3 = starke Schuppen mit Rötung
4 = Krusten mit Aufrichten der Haare.

Die Versuchsgruppen bestanden aus jeweils 6 Tieren. Die Kollektive wurden mathematisch auf Homogenität geprüft und nach Eliminierung signifikant abweichender Tiere wurden Mittelwert und Streuung für jede Substanz berechnet. Diese wurden in Beziehung gesetzt zu den Werten der nur mit 5 % Ölsäure in 70 %igem Ethanol behandelten Haut und daraus die Signifikanz der Hemmwirkung bestimmt. Das Ergebnis der Prüfung ist der nachstehenden Tabelle 1 zu entnehmen.

**T a b e l l e 1**

| Wirkstoff | Hautdickenmessung Hemmung % | (sign.)x) | Hautschuppenmessung Hemmung % | (sign.)x) |
|---|---|---|---|---|
| A | 15 | + | 100 | + |
| B | 10 | | 50 | + |

x) signifikant mit 95 %iger Wahrscheinlichkeit. Durch das Ergebnis der Versuche wurde die gute Antischuppenwirkung der erfindungsgemäß zu verwendenden Alkoxybenzoylaminoalkansäuren bzw. deren Derivaten vollauf bestätigt.

Nachstehend werden einige Beispiele für Haarbehandlungsmittel mit Antischuppenwirkung aufgeführt.

Beispiel 1 Alkoholisches Antischuppenhaarwasser

| | | |
|---|---|---|
| Produkt B | 3,0 | Gew.-Teile |
| Parfüm | 0,5 | " |
| Ethylalkohol 96 %ig | 67,0 | " |
| Wasser | 29,5 | " |

Beispiel 2 Haarfestlegemittel mit Antischuppenwirkung

| | | |
|---|---|---|
| Copolymerisat aus 60 Gew.-% Vinylpyrrolidon 40 Gew.-% Vinylacetat | 2,0 | Gew.-Teile |
| Sorbitanmonolaurat | 0,4 | " |
| Pflanzenextrakt | 0,5 | " |
| Parfüm | 0,6 | " |
| Produkt A | 2,5 | " |
| Isopropanol | 34,0 | " |
| Wasser | 60,0 | " |

/10

**Beispiel 3** Antischuppenshampoo

| | | |
|---|---|---|
| Natriumlaurylethersulfat 27 % Aktivsubstanz | 50,0 | Gew.-Teile |
| Kokosfettsäurediethanolamid | 2,0 | " |
| Polydiol 400 | 0,5 | " |
| Parfümöl | 0,3 | " |
| Kosmetikfarbstoff blau 0,1 %ig | 3,0 | " |
| Natriumalginat | 2,0 | " |
| Produkt B | 1,0 | " |
| Wasser | 41,2 | " |

**Beispiel 4** Antischuppenshampoo

| | | |
|---|---|---|
| Natriumlaurylethersulfat 27 % Waschaktivsubstanz | 63,0 | Gew.-Teile |
| Kokosfettsäurediethanolamid | 2,0 | " |
| Ethylenglykolstearinsäureester | 1,0 | " |
| Natriumchlorid | 2,0 | " |
| Proteinhydrolysat | 0,5 | " |
| Parfümöl | 0,5 | " |
| Produkt A | 2,0 | " |
| Wasser | 29,0 | " |

**Beispiel 5** Haarspülmittel mit Antischuppenwirkung

| | | |
|---|---|---|
| Wollfett | 1,0 | Gew.-Teile |
| Paraffinöl | 1,0 | " |
| Glyceridgemisch | 2,0 | " |
| Isopropylmyristat | 2,0 | " |
| Cetyl-stearylalkohol | 0,4 | " |
| Glycerin | 10,0 | " |
| Cetylpyridiniumchlorid | 0,5 | " |
| Natriumalginat | 0,2 | " |
| Parfümöl | 0,5 | " |
| Produkt B | 2,0 | " |
| Wasser | 80,4 | " |

2011845

Beispiel 6 Haarkurmittel mit Antischuppenwirkung

| | | |
|---|---|---|
| Wollfett | 1,0 | Gew.-Teile |
| Paraffinöl | 1,0 | " |
| Cetyl-stearylalkohol | 1,0 | " |
| Glyceridgemisch | 7,0 | " |
| Isopropylmyristat | 1,0 | " |
| Glycerin | 10,0 | " |
| Sorbitanmonolaurat | 1,0 | " |
| Pflanzenextrakt | 1,0 | " |
| Cetylpyridiniumchlorid | 0,5 | " |
| Natriumalginat | 0,2 | " |
| Parfümöl | 0,3 | " |
| Produkt A | 3,0 | " |
| Wasser | 73,0 | " |

Beispiel 7 Antischuppenshampoo

| | | |
|---|---|---|
| Cetylstearylalkohol mit ca. 40 Mol EO | 17,0 | Gew.-Teile |
| Kokosfettsäurediethanolamid | 2,0 | " |
| Ethylenglykolstearinsäureester | 1,0 | " |
| Cetylpyridiniumchlorid | 0,5 | " |
| Proteinhydrolysat | 0,5 | " |
| Produkt B | 2,0 | " |
| Parfümöl | 0,5 | " |
| Wasser | 76,5 | " |

**Beispiel 8** Antischuppenshampoo

| | | |
|---|---|---|
| Natriumlaurylethersulfat 27 % Waschaktivsubstanz | 36,0 | Gew.-Teile |
| Cetylstearylalkohol mit ca. 30 Mol EO | 7,5 | " |
| Kokosfettsäurediethanolamid | 2,0 | " |
| Ethylenglykolstearinsäureester | 1,0 | " |
| Natriumchlorid | 1,5 | " |
| Proteinhydrolysat | 0,5 | " |
| Parfümöl | 0,5 | " |
| Produkt A | 1,0 | " |
| Wasser | 50,0 | " |

An die Stelle der Produkte A und B können in den Beispielen für Haarbehandlungsmittel auch die anderen vorstehend genannten Alkoxy-benzoylaminoalkansäuren bzw. deren Derivate treten.

"Haarbehandlungsmittel mit Antischuppenwirkung"

## Patentansprüche

1. Haarbehandlungsmittel mit Antischuppenwirkung, dadurch gekennzeichnet, daß es in einem geeigneten Trägermaterial Alkoxy-benzoylaminoalkansäuren bzw. deren Derivate der allgemeinen Formel

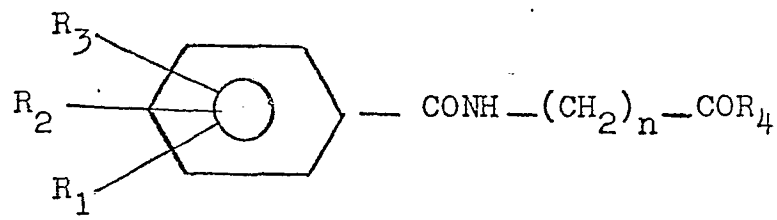

in der $R_1$ für eine Alkoxygruppe mit 1 - 4 C-Atomen, vorzugsweise eine Methoxygruppe, in der 3- oder 4-Position, $R_2$ und $R_3$ unabhängig voneinander für ein Wasserstoffatom, einen Alkylrest oder eine Alkoxygruppe mit 1 - 4 C-Atomen oder eine Hydroxygruppe, $R_4$ für eine Hydroxygruppe, eine Alkoxygruppe oder eine gegebenenfalls substituierte Aminogruppe und n für die Zahlen 1 - 6 stehen, in einer Menge von 0,01 bis 5 Gewichtsprozent, vorzugsweise 0,5 bis 3 Gewichtsprozent, bezogen auf das gesamte Mittel, enthält.

2. Haarbehandlungsmittel mit Antischuppenwirkung nach Anspruch 1, dadurch gekennzeichnet, daß es neben den Alkoxy-benzoylaminoalkansäuren bzw. deren Derivaten als tensidische Komponente Fettalkohol-polyalkylenoxid-Addukte, vorzugsweise solche von Fettalkoholen der Kettenlängen mit 12 - 18 Kohlenstoffatomen mit 5 - 40 Mol Ethylenoxideinheiten, enthält.